**Europäisches Patentamt**

**European Patent Office**

**Office européen des brevets**

(19)

(11) Veröffentlichungsnummer : **0 334 349 B1**

(12)

# EUROPÄISCHE PATENTSCHRIFT

(45) Veröffentlichungstag der Patentschrift :
**19.02.92 Patentblatt 92/08**

(51) Int. Cl.⁵ : **A61M 31/00,** A61M 35/00,
A61M 5/315

(21) Anmeldenummer : **89105231.8**

(22) Anmeldetag : **23.03.89**

(54) Vorrichtung zur dosierten Verabreichung eines flüssigen Arzneimittels.

(30) Priorität : **25.03.88 DE 3810262**

(43) Veröffentlichungstag der Anmeldung :
**27.09.89 Patentblatt 89/39**

(45) Bekanntmachung des Hinweises auf die
Patenterteilung :
**19.02.92 Patentblatt 92/08**

(84) Benannte Vertragsstaaten :
**AT BE CH DE ES FR GB GR IT LI LU NL SE**

(56) Entgegenhaltungen :
**US-A- 4 127 126**

(73) Patentinhaber : **HENNING BERLIN GmbH
CHEMIE- UND PHARMAWERK
Komturstrasse 58-62
W-1000 Berlin 42 (DE)**

(72) Erfinder : **Borchard, Hans-Jürgen
Holbeinstrasse 53
W-1000 Berlin 45 (DE)**

(74) Vertreter : **Andrae, Steffen, Dr. et al
Balanstrasse 55
W-8000 München 90 (DE)**

**Beschreibung**

Die Erfindung betrifft eine Vorrichtung zur dosierten Verabreichung eines flüssigen Arzneimittels nach dem Oberbegriff des Anspruchs 1, insbesondere zur intranasalen Verabreichung unter Zerstäuben eines solchen Arzneimittels.

Ein Beispiel einer solchen Vorrichtung ist in US-A-4 127 126 beschrieben.

Die Verabreichung von pharmazeutischen Wirkstoffen erfolgt üblicherweise durch die Verabreichung von Arzneimittelformen, die eine Einheitsdosis des jeweiligen Wirkstoffs enthalten. Wirkstoffe, die injiziert werden müssen, werden meist in Form von Ampullen auf den Markt gebracht, die eine zu verabreichende Dosis enthalten. Derartige Dosierungsformen haben den Vorteil, daß eine wiederholte Entnahme aus einem größeren Arzneimittelvorrat und anschließende Einzeldosierung vermieden wird, die aus hygienischen Gründen wenig wünschenswert oder sogar bedenklich erscheint. Bei flüssigen Arzneimitteln, die als solche direkt an einen Patienten verabreicht werden, bereitet jedoch die Vermarktung verabreichungsgerechter Einzeldosen größere Probleme, da es i. d. R. erforderlich ist, gleichzeitig Vorrichtungen mitzuliefern, die die Verabreichung technisch ermöglichen. Dadurch erhöhen sich jedoch die Kosten einer Einzeldosis so, daß der Vertrieb zahlreicher Medikamente in Einzeldosisform praktisch unmöglich gemacht wird.

Das gilt insbesondere auch für die intranasale Verabreichung von flüssigen Arzneimitteln, die dadurch verabreicht werden, daß ein Arzneimittel durch einen Sprühkopf in die Nase versprüht wird, wobei üblicherweise eine gleichmäßige Verteilung des Arzneimittels auf beide Nasenlöcher erwünscht ist. Da Sprühvorrichtungen relativ aufwendige Vorrichtungen sind, sind diese i. d. R. für eine mehrmalige Verwendung konzipiert. Insbesondere bei Arzneimitteln, die beispielsweise aus diagnostischen Zwecken nur ein einziges Mal intranasal an einen bestimmten Patienten verabreicht werden sollen, ist jedoch eine für eine Mehrfachverwendung bestimmte Sprühflasche nachteilig. Man kann zwar mit auswechselbaren Sprühköpfen oder entsprechen den Aufsätzen arbeiten; wenn es sich jedoch um ein in der ärztlichen Praxis relativ selten benötigtes Medikament handelt, bleibt auch dann der Nachteil, daß ein Arzt für gelegentliche Einzelanwendungen einen großen Vorrat des Arzneimittels benötigt. Ein derartiger Fall ist beispielsweise bei der Diagnostik von Schilddrüsenerkrankungen gegeben, wo zu diagnostischen Zwecken TRH (Thyreothropin-Releasing-Hormon) als Einzeldosis an einen Patienten verabreicht werden muß. Der durch die TRH-Dosis bewirkte Anstieg des TSH-Serumspiegels (Thyreothropin-Serumspiegels) ist diagnostisch zur Bestimmung verschiedener Schilddrüsenzustände signifikant.

Aus verschiedenen Gründen, z. B. der Verträglichkeit, ist die intranasale Verabreichung der gewünschten Testdosis besonders wünschenswert. Da die Testdosis jedoch nur ein einziges Mal, und dabei auch noch möglichst gleichmäßig auf beide Nasenlöcher verteilt, verabreicht werden soll, stellt sich das Problem, wie eine derartige Testdosis in einer für diese Einzeldosis geeigneten Sprühvorrichtung so in den Handel gebracht werden kann, daß die Sprühvorrichtung für die Einmalverwendung nicht zu einer übermäßigen Verteuerung des Präparats führt. Überschlagsmäßige Abschätzungen haben nämlich ergeben, daß die Verwendung speziell entwickelter Mini-Sprühvorrichtungen unter Berücksichtigung der Herstellungs- und Werkzeugkosten wirtschaftlich nachteilig ist und außerdem derartige Spezial-Sprühvorrichtungen, die eine dosierte Verabreichung ermöglichen, unter Berücksichtigung der insgesamt sehr kleinen zu verabreichenden Mengen relativ kompliziert gestaltet werden müssen.

Es ist Aufgabe der vorliegenden Erfindung, eine Vorrichtung zur Verabreichung eines flüssigen Arzneimittels, insbesondere zur intranasalen Verabreichung eines flüssigen Arzneimittels durch Versprühen von zwei gleichen Dosen, zu schaffen, die einerseits eine zuverlässige dosierte Verabreichung ermöglicht, andererseits jedoch aufgrund der Verwendung existierender billiger Massenartikel nur geringe Kosten verursacht.

Diese Aufgabe wird durch eine Vorrichtung zur dosierten Verabreichung eines flüssigen Arzneimittels mit den Merkmalen gemäß Anspruch 1 gelöst.

Vorteilhafte Ausgestaltungen sind den Unteransprüchen zu entnehmen.

Die erfindungsgemäße Vorrichtung greift soweit als möglich auf existierende billige Massenartikel, nämlich Injektionsspritzen und Zerstäuberköpfe zurück, die innerhalb einer einzigen Vorrichtung so miteinander kombiniert werden, daß einerseits die zusätzlichen Herstellungskosten möglichst gering gehalten werden, andererseits jedoch eine zuverlässig funktionierende Vorrichtung erhalten wird.

Die zu verabreichende Dosis, und zwar insbesondere in zwei Portionen zu verabreichende Dosis, wird dabei in Form einer mit dieser Dosis gefüllten üblichen Injektionsspritze in den Handel gebracht, die so mit weiteren Teilen zu der erfindungsgemäßen Vorrichtung kombiniert ist, daß ein sicherer Vertrieb dieser Injektionsspritze mit gezogenem Spritzenkolben möglich ist und der Inhalt der Spritze auf einfache, narrensichere Weise effektiv in Form zweier gleicher Portionen versprüht werden kann.

Zu diesem Zwecke wird die gefüllte Injektionsspritze einerseits in einem Röhrchen angeordnet, das ein seitliches Abknicken des Spritzenkolbens und unbeabsichtigtes Eindrücken dieses Spritzenkolbens verhindert, das jedoch gleichzeitig so gestaltet ist, daß im gewünschten Moment zum Zwecke der Verabreichung der Spritzenkolben eingedrückt werden kann. Vorzugsweise wird dieser Spritzenkolben dabei in mindestens zwei Schritten eingedrückt, die das Versprühen des Spritzeninhalts in Form von mindestens zwei entsprechenden Portionen bewirken.

Diese Eigenschaften werden vorzugsweise dadurch erreicht, daß die gefüllte Spritze in einem das Abknicken verhindernden Rohr angeordnet wird, das aus mindestens zwei teleskopartig aneinanderschiebbaren Rohrstücken besteht, die während des Vertriebs und der Lagerung so arretiert sind, daß keine Betätigung der Vorrichtung möglich ist, während im Bedarfsfall die Sperre einfach gelöst werden kann und das Rohr in Längsrichtung stufenweise zusammengedrückt werden kann.

Nachfolgend wird die Erfindung unter Bezugnahme auf die Zeichnungen anhand von bevorzugten Ausführungsformen noch näher erläutert.

Es zeigen:

Fig. 1 eine schematisiert dargestellte Gesamtansicht einer Ausführungsform einer erfindungsgemäßen Vorrichtung;

Fig. 2 einen Schnitt durch die erfindungsgemäße Vorrichtung ohne den nicht dargestellten Sprühkopf;

Fig. 3a-c schematische Detailansichten von denjenigen Vorrichtungselementen, die die stufenweise Betätigung gewährleisten, nämlich

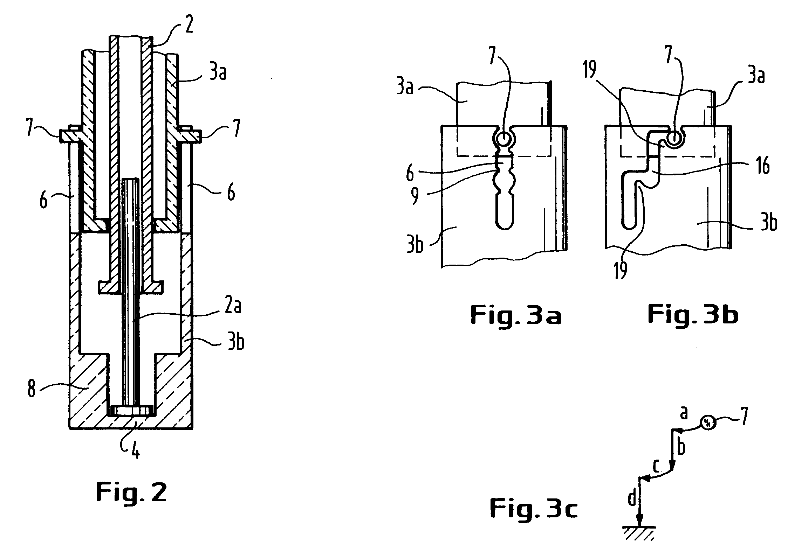

Fig. 3a einen in einem geraden Schlitz gleitenden Stift, von dem bestimmte Stellungen durch elastisches Einschnappen arretiert werden;

Fig. 3b die bevorzugte Ausführungsform, bei der ein Stift in einem stufenweise ausgebildeten Schlitz gleitet, und

Fig. 3c eine schematische Darstellung der Folge von Dreh- und Vorschubbewegungen, die zur Betätigung einer Vorrichtung mit den Elementen gemäß Fig. 3b erforderlich sind.

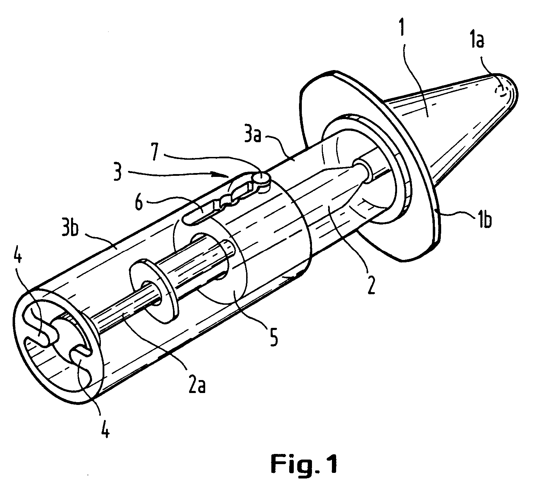

Bezug nehmend auf Fig. 1 besteht eine erfindungsgemäße Vorrichtung aus drei Grundelementen. Das erste ist der Ausgabekopf 1, der üblicherweise ein Sprühkopf zum Verstäuben eines Arzneimittels ist und eine Sprühdüse 1a und einen Kragen 1b aufweist, der das Festhalten des Ausgabekopfes 1 zwischen zwei Fingern erleichtert. Das zweite Grundelement ist eine Injektionsspritze 2 mit dem üblichen zugehörigen Spritzenkolben 2a. Diese Injektionsspritze 2 ist so dicht mit dem Ausgabekopf 1 verbunden, daß ein beim Eindrücken des Spritzenkolbens 2a in der Injektionsspritze 2 entwickelter Druck ohne Verlust an der Sprühdüse 1a wirksam wird. Zum Aufbau des erforderlichen Drucks hat es sich dabei als Vorteilhaft erwiesen, die Injektionsnadel auf der Injektionsspritze 2 zu belassen und den Sprühkopf um die Injektionsnadel und den Nadelansatz der Injektionsspritze 2 herum dicht anliegend anzuordnen.

Eine aus dem Ausgabekopf 1 und der Injektionsspritze 2 bestehende Vorrichtung ermöglicht zwar bereits ein Zerstäuben des Inhalts der Injektionsspritze, jedoch keine zuverlässige Portionierung und bildet außerdem eine für ein Handelsprodukt zu zerbrechliche Struktur. Für die erfindungsgemäße Vorrichtung ist daher als drittes Grundelement noch ein Rohr 3 erforderlich, das die Injektionsspritze mit dem gezogenen Spritzenkolben gegen Knickbelastungen sowie in axialer Richtung wirkende Krafteinwirkungen zum falschen Moment schützt. Der einfachste Fall bestünde darin, die Injektionsspritze in einem am Sprühkopf befestigten Röhrchen anzuordnen, das vor Gebrauch von der Vorrichtung entfernt wird. Das schrittweise Eindrücken des Spritzenkolbens könnte dann durch an der Spritze oder auf dem gezogenen Teil des Spritzenkolbens angeordneten Anschlägen gesichert werden, beispielsweise in Form abnehmbarer Röhrchen.

Gemäß der vorliegenden Erfindung wird jedoch der elegantere Weg beschritten, das Rohr 3 aus teleskopartig ineinandergreifenden Rohrteilen aufzubauen, und zwar vorzugsweise aus nur zwei Rohrteilen 3a und 3b. Sollte es aus irgendwelchen speziellen Gründen wünschenswert erscheinen, können jedoch ohne weiteres auch mehr derartige Rohrteile verwendet werden. Eines der Rohrteile 3a, das im Falle der dargestellten Ausführungsform das Rohrteil mit dem geringsten Durchmesser ist, ist ausreichend fest mit dem Ausgabekopf 1 verbunden. Das Ende dieses Rohrteils 3a ist in den Hohlraum des zweiten Rohrteils 3b eingeschoben, und zwar so, daß das Rohrteil 3b auf der Außenrohrfläche des Rohrteils 3a gleiten kann. Am Ende des Rohrteils 3b ist dieses geschlossen oder wenigstens mit Anschlägen 4 versehen, die gewährleisten, daß beim Verschieben des Rohrteils 3b gleichzeitig der Spritzenkolben 2a mit verschoben wird, das heißt, die Injektionsspritze betätigt wird. Im einfachsten bevorzugten Falle ist das Rohrteil 3b mit einem Boden 4 versehen, der die Funktion des Anschlags erfüllt. Um eine einfache Montage der gesamten Vorrichtung zu gewährleisten und ein Verkanten der Injektionsspritze während der Betätigung der Vorrichtung zu vermeiden, weisen die Rohrteile 3a und 3b geeignete Führungen auf, die eine koaxiale Anordnung der Injektionsspritze gewährleisten. In der gezeigten bevorzugten Ausführungsform wird dabei eine dieser Führungen, und zwar die im Rohrteil 3a, dadurch gebildet, daß das Rohrteil 3a ebenfalls einen Boden aufweist, in dem allerdings eine Durchgangsöffnung ausgeführt ist, durch die ohne übermäßiges Spiel der Kolben der Injektionsspritze hindurchgesteckt werden kann. Vorzugsweise weist auch das Rohrteil 3b noch eine Führung auf, wobei sich eine Führung 8, wie sie in Fig. 2 erkennbar ist, als vorteilhaft herausgestellt hat. Diese Führung 8 besteht darin, daß im verdickten Boden 4 des Rohrteils 3b eine zentrale Blindbohrung ausgeführt ist, die paßgenau das freie Ende des Spritzenkolbens aufnimmt und dieses während der Betätigung der Vorrichtung hält.

Ein teleskopartig zusammendrückbares Rohr muß vor Gebrauch gegen ein unbeabsichtigtes Zusammendrücken gesichert sein. Diese Sicherung kann durch entfernbare Sicherungselemente in Form von z. B. Klebestreifen gewährleistet sein, wird jedoch vorzugsweise von den gleichen Elementen übernommen, die die Größe der aus der Vorrichtung abgegebenen Portionen regeln. Diese Elemente sind vorzugsweise Vorsprünge und Vertiefungen, die an den beiden Rohrteilen angeordnet sind und im gewünschten Sinne zusammenwirken. Die Vorsprünge können dabei Stifte oder Knöpfe sein, die ggf. auch federbeaufschlagt sein können und die mit Schlitzen oder Nuten im anderen Teil zusammenwirken. Beispielsweise können elastische Knöpfe vorgesehen sein, die eingedrückt werden müssen, damit ein Vorschub des beweglichen Rohrteils 3b möglich ist. Mechanismen, wie sie z. B. von Schirmen bekannt sind, sind grundsätzlich verwendbar. Ferner ist es möglich, die Vorsprünge 7 und Aussparungen 6 so zu gestalten, wie in Fig. 3a dargestellt ist. In diesem Falle stellt die Aussparung einen geraden Schlitz dar, in dem ein Stift 7 gleiten kann. Stift 7 und Aussparung 6 bilden somit auf jeden Fall eine Führung für den geraden Vorschub des beweglichen Rohrteils 3b. Um bestimmte Stellungen des Rohrteils 3b zu fixieren, können in dem Schlitz 6 Nasen angeordnet sein, die den Stift 7 zwischen sich fangen, wenn er durch den Schlitz gleitet.

Eine solche Ausführungsform kann jedoch bei einer zu großen Krafteinwirkung dazu führen, daß das bewegliche Rohrteil 3b ungewollt zu weit eingedrückt wird. Das ist bei der bevorzugten Ausführungsform gemäß Fig. 3b jedoch ausgeschlossen. In diesem Falle ist der Schlitz 16 stufenförmig ausgestaltet. In der Ausgangsstellung wird der Stift 7 in einer Erweiterung des Schlitzes 16 gehalten. Gegen ein Herausziehen aus dem Schlitz ist er durch eine eine Verengung des Schlitzes bildende, elastisch überwindbare Nase gehalten. Ein Eindrücken des Rohrteils 3b wird durch eine Stufe sicher verhindert. Um die Vorrichtung betätigen zu können, muß, wie in Fig. 3c schematisch dargestellt ist, das Rohrteil 3b gegen das Rohrteil 3a gedreht werden, wobei vorzugsweise ein Widerstand in Form einer elastischen Nase 19 zu überwinden ist. Diese Drehung (a in Fig. 3c) ermöglicht anschließend ein Eindrücken des Rohrteils 3b über die Länge des geraden Schlitzabschnitts bis zur nächsten Stufe (3b in Fig. 3c). Eine Wiederholung dieser Bewegungen (c und d in Fig. 3c) ermöglicht dann ein vollständiges Aufschieben des Rohrteils 3b und damit gleichzeitig vollständiges Eindrücken des vorher gezogenen Spritzenkolbens. Auf diese Weise wird der Inhalt des Spritzenkolbens in zwei gleichen Portionen versprüht, deren Größe durch die Länge der geraden Abschnitte des stufenförmigen Schlitzes 16 vorgegeben ist. Selbstverständlich ist ein Versprühen in mehr Portionen möglich, wenn mehr Stufen vorgesehen werden.

Die Erfindung wurde vorstehend unter Bezugnahme auf konkrete Ausführungsbeispiele geschildert. Insbesondere was die Gestaltung des teleskopartig zusammendrückbaren Rohres 3 angeht, sind jedoch zahlreiche, für den Fachmann offensichtlich äquivalente Ausführungsformen denkbar. So können die Rohrteile 3a und 3b auch so angeordnet werden, daß das hintere Rohrteil den kleineren Durchmesser aufweist. Stifte und Schlitze können ebenfalls vertauscht angeordnet werden, und für die koaxiale Halterung der Injektionsspritze in den Röhrchen sind zahlreiche andere Ausführungsformen der Führungselemente denkbar, die außerdem auch noch zusätzlich zu den geschilderten verwendet werden können.

Als Sprühköpfe können handelsübliche, für die intranasale Verabreichung von flüssigen Arzneimitteln bestimmte Sprühköpfe verwendet werden. Derartige Sprühköpfe werden normalerweise in Verbindung mit einer Pumpe verwendet und sind einfache Kunststoff-Spritzgußteile mit einem engen zentralen Flüssigkeitskanal und einer Vernebelungsdüse. Derartige Sprühköpfe können beispielsweise bei der Firma Ing. Erich Pfeiffer GmbH & Co. KG, D-7760 Radolfszell, Bundesrepublik Deutschland, bezogen werden. Sie können gegebenenfalls etwas abgewandelt sein, damit die Spritzennadel dicht vom Flüssigkeitskanal umschlossen wird und genau bis in den Bereich unter der Vernebelungsdüse reicht.

Obwohl die vorliegende Erfindung für die portionsweise intranasale Verabreichung eines bestimmten Präparats gemacht wurde, sind ohne weiteres auch andere Einsatzmöglichkeiten denkbar. So kann eine erfindungsgemäße Vorrichtung auch einfach zum Vertrieb gefüllter Injektionsspritzen für die verschiedensten Zwecke verwendet werden, wobei in diesem Falle der Ausgabekopf durch eine einfache Schutzkappe zu ersetzen wäre. Eine entsprechende Gestaltung der Ausgabeöffnung vorausgesetzt, kann die Vorrichtung auch für die tropfenweise Verabreichung von Medikamenten, z. B. antibiotikahaltige Augentropfen, ausgestaltet werden. Ferner ist ein Versprühen nicht nur in die Nasenöffnungen möglich, sondern auch in beliebige andere Körperöffnungen, z. B. den Rachenraum. Soweit für derartige Verwendungszwecke die wesentlichen Merkmale einer erfindungsgemäßen Vorrichtung (siehe Anprüche) verwendet werden, sollen derartige Abwandlungen als Benutzungen der geschützten Vorrichtung angesehen werden.

**Patentansprüche**

1. Vorrichtung zur dosierten Verabreichung eines flüssigen Arzneimittels, versehen mit einem Ausgabekopf (1) an dem sich eine Ausgabedüse (1a) befindet und mit einer Injektionsspritze (2), wobei das nadelseitige Ende der Injektionsspritze dicht mit dem Ausgabekopf (1) verbunden ist, dadurch gekennzeichnet, daß die Vorrichtung ein Röhrchen (3) aufweist, das mit dem Ausgabekopf (1) verbunden ist, in dem die gefüllte Injektionsspritze (2) mit gezogenem Spritzenkolben (2a) angeordnet ist.

2. Vorrichtung nach Anspruch 1, dadurch gekennzeichnet, daß das Röhrchen (3) lösbar mit dem Ausgabekopf (1) verbunden ist.

3. Vorrichtung nach einem der Ansprüche 1 oder 2, dadurch gekennzeichnet, daß der Ausgabekopf (1) ein Sprühkopf mit einer Sprühdüse für die intranasale Verabreichung eines flüssigen Arzneimittels ist.

4. Vorrichtung nach einem der Ansprüche 1 bis 3, dadurch gekennzeichnet, daß das Röhrchen (3) aus mindestens zwei teleskopartig ineinanderschiebbaren Rohrteilen (3a, 3b) besteht,
   – von denen ein erstes Rohrteil (3a) mit dem Ausgabekopf (1) verbunden ist,
   – und ein anderes Rohrteil (3b) einen Anschlag (4) für das Ende des Spritzenkolbens (2a) aufweist, so daß dieser beim teleskopartigen Ineinanderschieben der Rohrteile (3a, 3b) eingedrückt wird,
   – und die Rohrteile (3a, 3b) mit zusammenwirkenden Aussparungen (6) und Vorsprüngen (7) versehen sind, die den gestreckten Zustand des Röhrchens (3) sowie vorzugsweise mindestens eine Zwischenstellung vor dem völlig eingedrückten Zustand fixieren.

5. Vorrichtung nach Anspruch 4, dadurch gekennzeichnet, daß mindestens eines der Rohrteile (3a, 3b) in seinem Inneren eine Führung (5, 8) aufweist, die eine koaxiale Anordnung der Injektionsspritze (2) im Röhrchen (3) gewährleistet.

6. Vorrichtung nach Anspruch 5, dadurch gekennzeichnet, daß eine der Führungen (5) als mit einer zentralen Durchgangsöffnung versehener Boden des Rohrteils (3a) mit dem kleinsten Durchmesser ausgebildet ist.

7. Vorrichtung nach Anspruch 5 oder 6, dadurch gekennzeichnet, daß eine weitere Führung (8) vorgesehen ist, die im Bereich des Anschlags (4) ausgebildet ist und das Ende des Spritzenkolbens (2a) in koaxialer Anordnung fixiert.

8. Vorrichtung nach einem der Ansprüche 3 bis 7, dadurch gekennzeichnet, daß die Aussparungen (6) die Form mindestens eines Längsschlitzes oder einer Längsnut aufweisen, die in einem der Rohrteile (3b) ausgeführt sind, und daß die Vorsprünge (7) in derartigen Aussparungen (6) gleitende Stifte sind, wobei die End- und Zwischenstellung durch elastisch überwindbare Verengungen (9) des mindestens einen Schlitzes (6) fixiert werden.

9. Vorrichtung nach Anspruch 8, dadurch gekennzeichnet, daß der mindestens eine Schlitz (16) stufig ausgebildet ist.

10. Vorrichtung nach einem der Ansprüche 1 bis 9, dadurch gekennzeichnet, daß das Röhrchen (3) aus einem opaken und/oder klaren Kunststoffmaterial hergestellt ist.


**Claims**

1. A device for adminstering doses of a liquid drug, provided with a dispensing head (1) on which a dispensing nozzle (1a) is located and with an injection syringe (2), the needle end of the injection syringe being sealingly connected to the dispensing head (1), characterised in that the device comprises a small tube (3) which is connected to the dispensing head (1) in which the filled injection syringe (2) with the syringe plunger (2a) retracted is disposed.

2. A device according to claim 1, characterised in that the tube (3) is releasably connected to the dispensing head (1).

3. A device according to claim 1 or 2, characterised in that the dispensing head (1) is a spray head with a spray nozzle for the intra-nasal administration of a liquid drug.

4. A device according to any one of claims 1 to 3, characterised in that the tube (3) consists of at least two telescopically slidable parts (3a, 3b),
   – of which a first part (3a) is connected to the dispensing head (1),
   – and another part (3b) has an abutment (4) for the end of the syringe plunger (2a), so that the latter is pressed in on telescopic movement of the tube parts (3a, 3b),
   – and the tube parts (3a, 3b) are provided with cooperating recesses (6) and projections (7) which fix the extended state of the tube (3) and preferably at least one intermediate position before the fully pressed-in state.

5. A device according to claim 4, characterised in that at least one of the tube parts (3a, 3b) has a guide (5, 8) in its interior to ensure a coaxial arrangement of the injection syringe (2) in the tube (3).

6. A device according to claim 5, characterised in that one of the guides (5) is formed as a centrally pierced base of the smaller-diameter tube part (3a).

7. A device according to claim 5 or 6, characterised in that another guide (8) is provided which is formed in the region of the abutment (4) and fixes the end of the syringe plunger (2a) in a coaxial arrangement.

8. A device according to any one of claims 3 to 7, characterised in that the recesses (6) are in the form of at least one longitudinal slot or a longitudinal groove formed in one of the tube parts (3b) and in that the projections (7) are pins sliding in such recesses (6), the end and intermediate positions being fixed by resiliently passable constrictions (9) in the or each slot (6).

9. A device according to claim 8, characterised in that the or each slot (16) is of stepped construction.

10. A device according to any one of claims 1 to 9, characterised in that the tube (3) is formed from an opaque and/or clear plastic material.


## Revendications

1. Dispositif pour administrer de façon dosée un médicament liquide, pourvu d'une tête débitrice (1), sur laquelle se trouve une buse débitrice (1a) et d'une seringue d'injection (2), l'extrémité côté aiguille de la seringue d'injection étant reliée de façon étanche à la tête débitrice (1), caractérisé en ce que le dispositif présente un petit tube (3), relié à la tête débitrice (1), dans lequel est disposé la seringue d'injection (2) remplie, le piston de seringue étant tiré.

2. Dispositif selon la revendication 1, caractérisé en ce que le petit tube (3) est relié amovible à la tête débitrice (1).

3. Dispositif selon l'une des revendications 1 ou 2, caractérisé en ce que la tête débitrice (1) est une tête de pulvérisation, avec une buse de pulvérisation pour l'administration intranasale d'un médicament liquide.

4. Dispositif selon l'une des revendications 1 à 3, caractérisé en ce que le petit tube (3) se compose de deux parties de tube (3a,3b) susceptibles d'être insérées l'une dans l'autre de façon télescopique,
   – dont une première partie de tube (3a) est reliée à la tête débitrice (1),
   – et une autre partie de tube (3b) présente une butée (4), pour l'extrémité du piston de seringue (2a), de sorte que ce dernier est introduit sous pression lors de la rétraction télescopique des parties de tube (3a,3b),
   – et les parties de tube (3a,3b) sont pourvues d'évidements (6) et de saillies (7) qui coopèrent, en fixant l'état déployé du petit tube (3), ainsi que, de préférence, au moins une position intermédiaire avant d'arriver à l'état totalement rétracté.

5. Dispositif selon la revendication 4, caractérisé en ce qu'au moins l'une des parties de tube (3a,3b) présente intérieurement un guidage (5,8) qui assure une position axiale de la seringue d'injection (2) dans le petit tube (3).

6. Dispositif selon la revendication 5, caractérisé en ce que l'un des guidages (5) est réalisé sous forme d'un fond, pourvu d'un orifice de passage central de la partie de tube (3a) de plus petit diamètre.

7. Dispositif selon la revendication 5 ou 6, caractérisé en ce qu'est prévu un autre guidage (8), réalisé dans la zone de la butée (4) et fixant l'extrémité du piston de seringue (2a) en position axiale.

8. Dispositif selon l'une des revendications 3 à 7, caractérisé en ce que les évidements (6) présentent la forme au moins d'une fente allongée ou d'une rainure longitudinale, réalisés dans l'une des parties de tube (3b), et en ce que les saillies (7) sont des tiges coulissant dans les évidements (6) de ce genre, les positions finale et intermédiaire étant fixées au moyen de rétrécissements (9), susceptibles d'être surmontés, de la (au moins une) fente (6).

9. Dispositif selon la revendication 8, caractérisé en ce que la (au moins une) fente (16) est étagée.

10. Dispositif selon l'une des revendications 1 à 9, caractérisé en ce que le petit tube (3) est fabriqué à partir d'une matière synthétique opaque et/ou claire.

**Fig. 1**

**Fig.3a**

**Fig.3b**

**Fig.3c**

**Fig. 2**